# EUROPÄISCHE PATENTANMELDUNG

(11) **EP 4 806 504 A1**
(43) Veröffentlichungstag der Anmeldung: **16.09.2026**
(21) Anmeldenummer: 25217830.6
(22) Anmeldetag: 21.11.2025
(51) Int. Cl.: A61N 1/39, A61N 1/05

(54) **DEFIBRILLATOR**

(30) Priorität: 15.03.2025 DE 102025110017; 07.04.2025 DE 102025113660; 09.04.2025 DE 102025114064; 26.06.2025 DE 102025124883
(71) Anmelder: Mehnert, Walter, 85521 Ottobrunn (DE); Theil, Thomas, 82340 Feldafing (DE); Reimann, Rainer, 82386 Oberhausen (bei Peissenberg) (DE)
(72) Erfinder: Mehnert, Walter, 85521 Ottobrunn (DE); Theil, Thomas, 82340 Feldafing (DE); Reimann, Rainer, 82386 Oberhausen (bei Peissenberg) (DE)
(74) Vertreter: Kilian Kilian & Partner mbB

(57) **Zusammenfassung**

Die Erfindung betrifft einen Defibrillator (1) zur Implantation in einen Körper eines Lebewesens und zur Steuerung einer Herzaktivität des Herzens des Lebewesens, aufweisend:
einen Elektrodenabschnitt (2), der bestimmungsgemäß bei Implantation des Defibrillators derart angeordnet wird, dass ein über den Elektrodenabschnitt (2) abgegebener Stromstoß (ST) die Herzaktivität steuert
eine mit dem Elektrodenabschnitt (2) verbundene Schaltelektronik (3), die eingerichtet ist, den Defibrillator zu steuern und die Abgabe des Stromstoßes (ST) auszulösen;
mindestens einen elektrischen Energiespeicher (4), der eine Spannung zur Langzeitversorgung liefert, wobei
die Schaltelektronik (3) eingerichtet ist, die gelieferte Spannung zur Erzeugung einer Stoßspannung, mit der der Stromstoß (ST) zur Steuerung der Herzaktivität abgegeben wird, zu vervielfältigen; und
einen mit der Schaltelektronik (3) verbundenen Energieempfangsabschnitt (5), der derart eingerichtet ist, dass er Energie kontaktlos empfangen und an den Energiespeicher (4) zum Wiederaufladen des Energiespeichers (4) abgeben kann, wobei der Energieempfangsabschnitt (4) zumindest eine Spule (52) aufweist, die eingerichtet ist, die Energie zu empfangen und über einen Gleichrichter (51) an den Energiespeicher (4) abzugeben, wenn sie von einem magnetischen Wechselfeld, das ein externes Ladegerät erzeugt, durchsetzt wird.

## Beschreibung

Die Erfindung betrifft einen aufladbaren Defibrillator zur bevorzugt vollständigen Implantation in einen Körper eines Lebewesens und zur Steuerung einer Herzaktivität eines Herzens des Lebewesens. Insbesondere betrifft die Erfindung einen Defibrillator, der bevorzugt vollständig in den menschlichen Körper implantiert wird und der die Herzaktivität des menschlichen Herzens steuert.

Defibrillatoren sind im Stand der Technik allgemein bekannt. Neben externen Defibrillatoren, die ein Arzt oder Helfer in einem akuten Notfall extern an den Körper eines Patienten anlegt, um einen die Herzaktivität steuernden Stromstoß zu applizieren, existieren implantierbare Defibrillatoren.

Letztere verbleiben nach Implantation dauerhaft im Körper des Patienten, überwachen die Herzaktivität und geben bei Bedarf einen Stromstoß an das Herz des Patienten ab.

Die implantierten Defibrillatoren beziehen die für ihre Funktionsweise notwendige Energie aus einer Batterie, wobei die Elektronik so entworfen ist, dass der Energieinhalt der Batterie ausreicht, den entsprechenden Defibrillator bis zum Ableben des Patienten oder zumindest über Dekaden hinweg dauerhaft mit Energie zu versorgen. Sollte der Fall eintreten, dass der Energieinhalt der Batterie nicht ausreicht, erfolgt ein Auswechseln der Batterie durch Vornahme eines chirurgischen Eingriffs. Bereits dieser Umstand ist misslich.

Hinzukommt, dass Batterien für die Anwendung in Defibrillatoren elektrische Einschränkungen besitzen, die nur durch unerwünschte Maßnahmen zu kompensieren sind.

Der durch die bekannten Defibrillatoren applizierte Stromstoß erfolgt beispielsweise bei einer Spannung von +/-400V mit nicht unerheblichen Strömen. Zum einen liefern die Batterien solche Spannungen nicht, weshalb ein Transformator zur Erhöhung der von der entsprechenden Batterie gelieferten Spannung zum Einsatz kommt. Soll der Stromstoß gleichzeitig mit hoher Stromstärke erfolgen, erforderte das je nach Wicklungsverhältnis des Transformators einen Strom mehrerer Ampere auf der Primärseite des Transformators. Bekannte einsetzbare Batterien liefern solche Ströme nicht. Entnahmeraten (entnehmbare Stromstärke/Ladungsinhalt; Ampere/pro Amperestunde) bekannter guter Batterien liegen bei ca. 10 Ampere/pro Amperestunde.

Benötigte der Transformator auf seiner Sekundärseite 1 A bei 400 V dann ergibt das auf der Primärseite beispielsweise 100A bei 4V. In diesem Fall erforderte die Batterie eine Ladung von 33000 As. Dies entspricht heute einem Batterievolumen von 33 ccm. Eine Implantation einer solchen Batterie ist nahezu ausgeschlossen.

Um die erläuterte Problematik zu umgehen, besitzen die bekannten, implantierbaren Defibrillatoren Speicherkondensatoren, die über die Sekundärseite des Transformators auf dem erwähnten Spannungsniveau mit Strömen im mA-Bereich so lange aufgeladen werden, bis der entsprechende Speicherkondensator einen Stromstoß auf dem Spannungsniveau mit der erforderlichen Stromstärke abgeben kann.

Die erwähnten Speicherkondensatoren besitzen einerseits unerwünscht große Abmessungen. Anderseits stört die Ladezeit.

Eine Implantation der bekannten Defibrillatoren in das menschliche Herz ist bei den erläuterten Restriktionen auch aufgrund der resultierenden Baugröße ausgeschlossen.

Vor obigem Hintergrund ist es Aufgabe der Erfindung, einen verbesserten Defibrillator zu schaffen. Zumindest ist es Aufgabe der Erfindung einen zum Stand der Technik alternativen Defibrillator zu schaffen.

Diese Aufgabe(n) löst ein Defibrillator nach Anspruch 1. Weitere bevorzugte Ausführungsformen sind Gegenstand der abhängigen Ansprüche.

*Ein erfindungsgemäßer Defibrillator zur Implantation in einen Körper eines Lebewesens und zur Steuerung einer Herzaktivität des Herzens des Lebewesens beinhaltet:*
*einen Elektrodenabschnitt, der bestimmungsgemäß bei Implantation des Defibrillators derart angeordnet wird, dass ein über den Elektrodenabschnitt abgegebener Stromstoß die Herzaktivität steuert;*
*eine mit dem Elektrodenabschnitt verbundene Schaltelektronik, die eingerichtet ist, den Defibrillator zu steuern und die Abgabe des Stromstoßes auszulösen;*
*mindestens einen elektrischen Energiespeicher, der eine Spannung zur Langzeitversorgung liefert, wobei*
*die Schaltelektronik eingerichtet ist, aus der gelieferten Spannung eine Stoßspannung, mit der der Stromstoß zur Steuerung der Herzaktivität abgegeben wird, zu erzeugen, bevorzugt die gelieferte Spannung zur Erzeugung der Stoßspannung zu vervielfältigen; und*
*einen mit der Schaltelektronik verbundenen Energieempfangsabschnitt, der derart eingerichtet ist, dass er Energie kontaktlos empfangen und an den Energiespeicher zum Wiederaufladen des Energiespeichers abgeben kann, wobei der Energieempfangsabschnitt zumindest eine Spule aufweist, die eingerichtet ist, die Energie zu empfangen und über einen Gleichrichter an den Energiespeicher abzugeben, wenn sie von einem magnetischen Wechselfeld, das ein externes Ladegerät erzeugt, durchsetzt wird.*

Der Elektrodenabschnitt kann eine auf der Außenoberfläche des Defibrillatorgehäuses exponierte Elektrode aufweisen. Alternativ kann der Elektrodenabschnitt eine Kabelsonde aufweisen, die kabelgebunden von dem Defibrillatorgehäuse an einen bestimmten Ort innerhalb des Körpers läuft und dort der Stromstoß abgebbar ist.

Der erfindungsgemäße Defibrillator kann in verschiedenen Implantationsvarianten realisiert sein.

Gemäß einer bevorzugten ersten Implantationsvariante wird der Defibrillator unter der Haut neben einen Muskel oder in einen Muskel implantiert. Beispielsweise wird der Defibrillator in dieser Implantationsvariante unter/neben dem Brustbein oder unter/in den Brustmuskel implantiert.

Der Elektrodenabschnitt gemäß dieser ersten Implantationsvariante besitzt beispielsweise mindestens eine Kabelsonde, die von dem Defibrillatorgehäuse an einen geeigneten herznahen Ort geführt wird und von dort den Stromstoß abgibt. Die Kabelsonde berührt das Herz ergo nicht unmittelbar (sondern nur über dazwischenliegendes Gewebe mittelbar). Eine weitere Elektrode des Elektrodenabschnittes kann eine auf dem Defibrillatorgehäuse exponierte Elektrodenfläche oder eine weitere Kabelsonde sein. Auch das Defibrillatorgehäuse oder die weitere Kabelsode wird bestimmungsgemäß so angeordnet, dass die exponierte Elektrode oder die weitere Kabelsonde das Herz nicht unmittelbar berührt (sondern nur über dazwischenliegendes Gewebe mittelbar). Die Elektroden werden bestimmungsgemäß so angeordnet, dass sich das Herz in Bezug auf den Verlauf des elektrischen Feldes möglichst optimal im Feldverlauf befindet und bei Abgabe des Stromstoßes viel Feldwirkung erfährt. Diese erste Implantationsvariante wird im Folgenden bezeichnet mit "erste indirekte Implantationsvariante".

Alternativ besitzt der Elektrodenabschnitt gemäß dieser ersten Implantationsvariante bevorzugt mindestens eine Kabelsonde, die von den Defibrillatorgehäuse an eine Herzaußenwand geführt wird, oder mindestens eine, bevorzugt venöse, Gefäß-Kabelsonde, die von dem Defibrillatorgehäuse durch ein Gefäß ins Herz geführt sowie dort verankert wird. Eine weitere Elektrode des Elektrodenabschnittes kann eine auf dem Defibrillatorgehäuse exponierte Elektrodenfläche, eine weitere an die Herzaußenwand geführte Kabelsonde, oder eine weitere, bevorzugt venöse, Gefäß-Kabelsonde sein. Diese Implantationsvariante wird im Folgenden bezeichnet mit "zweite direkte Implantationsvariante".

Bevorzugt kann der Defibrillator gemäß einer "dritten bevorzugten kardialen Implantationsvariante" eine Herzkapsel sein, die bestimmungsgemäß vollständig ins Herz, beispielsweise in eine der Herzkammern implantiert wird. Eine entsprechende Verankerung erfolgt beispielsweise über eine von einer Außenoberfläche der Herzkapsel vorstehende Verankerungsspirale, die bestimmungsgemäß an entsprechender Stelle in das Herzgewebe gedreht wird. Bevorzugt besitzt die Herzkapsel noch eine weitere linear geführte Verankerung.

Der Elektrodenabschnitt der dritten kardialen Implantationsvariante ist beispielsweise durch auf der Außenoberfläche der Herzkapsel exponierte Elektrodenflächen gebildet.

Bevorzugt übernimmt die Verankerungsspirale und/oder die weitere linear geführte Verankerung eine Doppelfunktion, indem sie zusätzlich als eine der Elektroden fungiert bzw. fungieren. Eine oder beide der Elektrodenflächen sind demnach entbehrlich.

Der Umstand, dass der Energiespeicher ein wiederaufladbarer Energiespeicher ist, bringt mehrere Vorteile mit sich.

Beispielsweise kann der Defibrillator gemäß der dritten Implantationsvariante so klein gebaut werden, dass er in eine Herzkammer implantierbar ist. In der weiteren Implantationsvariante des Defibrillators besitzt die Herzkapsel ein Volumen von 0,5 cm³ bis 1,5 cm³, insbesondere von 0,8 cm³.

Auch in der ersten und zweiten Implantationsvariante lässt sich ein geringes Volumen des Defibrillators erzielen, indem das Defibrillatorgehäuse, in dem bis auf den Elektrodenabschnitt - der verständlicherweise außerhalb des Gehäuses angeordnet ist - die anspruchsgemäßen, genannten Komponenten aufgenommen sind, zwischen 4 cm³ bis 6 cm³, insbesondere 5 cm³ beträgt.

Außerdem sind Entnahmeraten C (1/h) von wiederaufladbaren Energiespeichern erheblich besser als von Batterien, sodass eine höhere Flexibilität besteht, Charakteristiken des Stromstoßes je nach Aufbau und Implantationsvariante geeignet zu wählen. Beispielsweise lassen sich trotz geringen Volumens aller Implantationsvarianten im Millisekunden-Bereich liegende Stromstöße hohen Ladungsinhalts realisieren.

Der erfindungsgemäße Defibrillator ist bevorzugt so konfiguriert, dass der Stromstoß beispielsweise eine Stromstärke zwischen 0,2 A und 1 A oder zwischen 0,5 A und 2A aufweist, sodass der Stromstoß im Millisekunden-Bereich ausreichend Ladungsinhalt besitzt.

Besonders bevorzugt kann der Ladungsinhalt in der zweiten direkten Implantationsvariante und der dritten Implantationsvariante als Herzkapsel auch niedriger ausfallen, beispielsweise von 3 bis 4 µAs mit 3ms bis 4ms und 1mA im Idealfall.

Beispielsweise ist der Energiespeicher ein elektrochemischer Akkumulator oder ein Festkörperakkumulator. Der Defibrillator kann mit einem einzigen Akkumulator auskommen, insbesondere in der dritten Implantationsvariante, in der der Defibrillator in direktem Kontakt mit dem Herz steht (innerhalb der Herzkammer). Durch diese räumliche Nähe kann die Stoßspannung und/oder die Stromstärke des Stromstoßes niedriger ausfallen, sogar im Grenzfall in der Größenordnung eines normalen Herzimpulses.

*Bevorzugt ist die Schaltelektronik des Defibrillators eingerichtet ist, die Stoßspannung bei Abgabe des Stromstoßes oder die Stoßspannung aufeinanderfolgender Stromstöße umzupolen, um so eine Stromrichtung des Stromstoßes zu ändern.*

Die Umpolung hat die Herzaktivität steuernde Vorzüge. Die Umpolung erfolgt beispielsweise durch eine Umpoleinrichtung, die beispielsweise durch eine Vielzahl an Schaltern aufgebaut ist. Die Schalter sind beispielsweise Transistoren, wie IGBTs oder MOSFETs.

*Bevorzugt beinhaltet der Defibrillator eine Steuer-, Verarbeitungs- und Speicherelektronik mit einer Kommunikationseinheit, die eingerichtet ist, zumindest mit dem externen Ladegerät zur Ausrichtung und*/*oder Veränderung des magnetischen Wechselfeldes zu kommunizieren, um eine für das Wiederaufladen erforderliche Zeit zu vermindern.*

Die Kommunikationseinheit kommuniziert mit dem Ladegerät per Funkverbindung, beispielsweise Bluetooth, Low Power Bluetooth oder einem anderen Kommunikationsstandard. Insbesondere kann die Kommunikationseinheit dem Ladegerät die räumliche Ausrichtung des implantierten bzw. des Energieempfangsabschnittes und/oder die Ladeperformance mitteilen. Das Ladegerät kann hieraus schließen, ob/wie es die Ausrichtung und/oder die Stärke des magnetischen Wechselfeldes ändern muss.

Die Erzeugung der ausgegebenen Stoßspannung kann ohne oder mit Vervielfachung der von den Energiespeichern 41 bis 45 gelieferten Spannung erfolgen oder auch unter Reduzierung der genannten Spannung erfolgen.

*Die Schaltelektronik ist allerdings bevorzugt eingerichtet, die gelieferte Spannung zur Erzeugung der Stoßspannung zu vervielfältigen.*

Diesbezüglich bestehen verschiedene Möglichkeiten.

*Bevorzugt weist der Defibrillator diesbezüglich auf: eine Vielzahl von elektrischen Energiespeichern, die die Spannung zur Langzeitversorgung liefern, wobei die Schaltelektronik eingerichtet ist, die Energiespeicher für das Wiederaufladen parallel zu schalten und, um die Vervielfachung der Spannung zumindest teilweise durchzuführen, in Reihe zu schalten.*

Für dieses Schalten zwischen Parallelschaltung und Reihenschaltung besitzt die Schaltelektronik eine Vielzahl von Schaltern, die wiederum beispielsweise Transistoren, wie IGBTs oder MOSFETs sein können.

Der Defibrillator hat beispielsweise vier bis fünf Energiespeicher. Die Energiespeicher sind bevorzugt jeweils elektrische Akkumulatoren, beispielsweise elektrochemische Akkumulatoren oder Festkörperakkumulatoren.

*In einer alternativen oder zusätzlichen Möglichkeit beinhaltet die Schaltelektronik des Defibrillators bevorzugt einen Transformator, der die zur Langzeitversorgung gelieferte oder teilweise vervielfachte Spannung zur Erzeugung der Stoßspannung vervielfacht.*

Ein Übertragungsverhältnis des Transformators kann unproblematisch hohe Werte aufweisen, sodass der Transformator die auf einer Primärseite des Transformators vorliegende, zur Langzeitversorgung gelieferte oder teilweise vervielfachte Spannung in hohe auf einer Sekundärseite des Transformators vorliegende Spannungen herauftransformieren kann, soweit die Ladungskapazität ausreicht. Selbst bei Stromstößen mit hohen Stromstärken im Amperebereich (bspw. 1A) und damit einhergehenden noch höheren Stromstärken auf der Primärseite des Transformators können insbesondere kleine Akkumulatoren die benötigten Ströme liefern.

Der Transformator benötigt auf der Primärseite eine Wechselspannung. *Bevorzugt ist der Defibrillator eingerichtet, dass die zur Langzeitversorgung gelieferte oder teilweise vervielfachte Spannung über einen Wechselrichter der Schaltelektronik an einer primärseitigen Spule des Transformators anliegt, und die Stoßspannung an einer sekundärseitigen Spule des Transformators ausgegeben wird.*

Die an der sekundärseitigen Spule ausgegebene Stoßspannung kann über einen Gleichrichter der Schaltelektronik gleichgerichtet werden.

*Bevorzugt ist der Defibrillator aufgebaut, dass die Spule des Energieempfangsabschnitts eine Doppelfunktion übernimmt, indem die Spule für das Wiederaufladen des*/*der Energiespeicher(s) eine Empfangsspule bildet und für die Vervielfachung der zur Langzeitversorgung gelieferten oder teilweise vervielfachten Spannung eine primärseitige Spule des Transformators bildet.*

*Bevorzugt ist der Defibrillator aufgebaut, wobei der Wechselrichter der Schaltelektronik ein bidirektionaler Wechsel-*/*Gleichrichter ist, der*
*(i) zum einen bei dem Wiederaufladen eine von der Empfangsspule ausgegebene Ladewechselspannung in eine Ladegleichspannung gleichrichtet und dem*/*den Energiespeicher(n) zuführt, und (ii) zum anderen bei Erzeugung der Stoßspannung die zur Langzeitversorgung gelieferte oder teilweise vervielfachte Spannung wechselrichtet und der primärseitigen Spule des Transformators zuführt.*

*Alternativ hierzu ist der Defibrillator aufgebaut, wobei die Schaltelektronik neben dem Wechselrichter, der bei Erzeugung der Stoßspannung die zur Langzeitversorgung gelieferte oder teilweise vervielfachte Spannung wechselrichtet und der primärseitigen Spule des Transformators zuführt, einen gesonderten Gleichrichter aufweist, der bei dem Wiederaufladen eine von der Empfangsspule ausgegebene Ladewechselspannung in eine Ladegleichspannung gleichrichtet und dem*/*den Energiespeicher(n) zuführt.*

Durch Vorsehen eines bidirektionalen Wechsel-/Gleichrichters oder getrennter Wechsel-/Gleichrichter ergeben sich Spielräum im Design des Defibrillators in Bezug auf Volumen und/oder Kosten.

*Bevorzugt ist der Defibrillator aufgebaut, wobei die Spule des Energieempfangsabschnittes einen magnetisch leitenden Kern, bevorzugt einen Ferrit, beinhaltet.*

Der magnetisch leitende Kern sorgt für ein Sammeln des von dem Ladegerät erzeugten magnetischen Wechselfeldes und damit für eine verbesserte Aufladung und bevorzugt auch für eine geringe Baugröße.

*Bevorzugt umschließt der Kern der Spule des Energieempfangsabschnittes einen Transformatorkern des Transformators.*

Dieser Aufbau und die Aufnahme des Transformatorkerns in den Kern der Spule des Energieempfangsabschnittes sind insbesondere dahingehend vorteilhaft, dass der Transformatorkern, der auch ein magnetisch gut leitendes Element ist, keine negativen Auswirkungen auf das Wiederaufladen hat, weil keine Gefahr besteht, dass der Transformatorkern das zum Wiederaufladen erzeugte magnetische Wechselfeld unbeabsichtigt aus der oder um die Spule des Energieempfangsabschnitt herum ablenkt.

*Der Transformator ist bevorzugt ein Kerntransformator, bevorzugt ein Ringkerntransformator oder Topfkerntransformator oder Hohlzylinderkerntransformator.*

Alternativ kann der magnetisch leitende Kern der Spule des Energieempfangsabschnittes gleichzeitig den Kern des Transformators bilden, insbesondere wenn die Spule des Energieempfangsabschnittes die erwähnte Doppelfunktion übernimmt.

*Bevorzugt ist der Defibrillator aufgebaut, wobei der Kern der Spule ein Gehäuse bildet, in dem die Steuer-, Verarbeitungs- und Speicherelektronik und*/*oder Schaltelektronik, und der*/*die Energiespeicher aufgenommen sind und auf das die Spule des Energieempfangsabschnittes gewickelt.*

Das die Spule tragende Gehäuse kann ein Defibrillatorgehäuse des Defibrillators bilden oder ein in dem Defibrillator aufgenommenes Innengehäuse bilden.

*Bevorzugt ist der Defibrillator aufgebaut, wobei das den Kern der Spule bildende Gehäuse einen Transformatorkern des Transformators bildet, und die die Stoßspannung ausgebende sekundärseitige Spule des Transformators auf das Gehäuse gewickelt ist und bevorzugt die Spule des Energieempfangsabschnittes trägt.*

Zwischen der Spule des Energieempfangsabschnittes und der sekundärseitigen Spule kann eine Isolierung, wie beispielsweise ein Isolierpapier/-folie angeordnet sein.

Die Spule des Energieempfangsabschnittes kann, wie erwähnt, eine Doppelfunktion übernehmen und neben der Funktion der für die Wiederaufladung des/der Energiespeicher(s) notwendigen Ladespule bzw. Empfangsspule auch die primärseitige Spule des Transformators bilden. D.h. während der Wiederaufladung induziert das magnetische Wechselfeld eine Wechselspannung in der Spule des Energieempfangsabschnittes, die daraufhin einen Ladestrom abgibt, der nach Gleichrichtung dem/den Energiespeicher(n) zugeführt wird. Wenn der Defibrillator nach Wiederaufladung in seinen Betrieb übergeht und bei Bedarf den Stromstoß abgibt, übernimmt die Spule die Funktion der primärseitigen Spule des Transformators, der die von dem/den Energiespeicher(n) zur Langzeitversorgung gelieferte Spannung nach Wechselrichtung zugeführt wird.

Insoweit können der Transformator und der Energieempfangsabschnitt mit zwei Spulen auskommen.

Alternativ können die Spulen des Transformators und des Energieempfangsabschnitts durch getrennte Spulen realisiert sein. D.h. der Defibrillator besitzt drei Spulen, die Spule des Energieempfangsabschnitts, die primärseitige Spule und die sekundärseitige Spule des Transformators.

*Bevorzugt ist der Defibrillator aufgebaut, wobei bei Auslösung durch die Schaltelektronik die Stoßspannung über den Elektrodenabschnitt ausgegeben wird und der Stromstoß aus dem*/*den Energiespeichern oder von der sekundärseitigen Spule des Transformators über den Elektrodenabschnitt zur Steuerung der Herzaktivität direkt, ohne Zwischenspeicherung, abgegeben wird oder direkt, lediglich unter Glättung, abgegeben wird.*

Diese direkte Abgabe des Stromstoßes aus dem/den Energiespeichern oder von der sekundärseitigen Spule des Transformators hat erhebliche Vorteile, die sich in dem Einsatz des wiederaufladbaren Energiespeichers begründen.

In allen Implantationsvarianten des Defibrillators können die Stromstöße mit hohen Stromstärken deshalb abgegeben werden, weil wiederaufladbare Energiespeicher mit hohen Entnahmeraten existieren und einsetzbar sind. Dies gilt selbst für die erste Implantationsvariante bei hohen Spannungen.

Der Energiespeicher ist bevorzugt ein elektrischer Akkumulator und in der ersten indirekten Implantationsvariante bevorzugt so aufgebaut, dass er beispielsweise zwischen 2000 As und 3000 As, bevorzugt 2500 As, installierten Ladungsinhalt besitzt. Der Energiespeicher ist in der ersten indirekten Implantationsvariante bevorzugt so aufgebaut, dass er beispielsweise einen elektrischen Strom von 10 A bis 25 A (Ampere), sogar auch von 10 A bis 100 A, liefern kann. Wenn der Energiespeicher beispielsweise aus der Vielzahl an Energiespeichern aufgebaut ist, liefert beispielsweise jeder Energiespeicher eine Spannung von 4V, hat einen Ladungsinhalt von 500 As und ist so aufgebaut, dass er 43mA pro As Ladungsinhalt liefern kann. Insoweit liegt der entnehmbare Strom in der genannten Größenordnung von 21,5 A, wobei sich bei beispielsweise vier/fünf Energiespeichern der Ladungsinhalt insgesamt auf 2000 As/ 2500 As beläuft.

Die Stoßspannung beträgt in der ersten indirekten Implantationsvariante bevorzugt +/-400 V. Die hierfür notwendige Vervielfachung erfolgt bevorzugt durch den erwähnten Transformator und bevorzugt durch das In-Reihe-Schalten von beispielsweise fünf Energiespeichern, die die aus der Reihenschaltung resultierende Spannung, von beispielsweise 20V an die primärseitige Spule des Transformators liefern. Der Stromstoß erfolgt bevorzugt mit 1A bei +/-400V über eine Dauer von 5ms bis 10ms.

Das Übertragungsverhältnis des Transformators beträgt folglich 20, sodass ein Strom von 20 A auf der Primärseite des Transformators erforderlich ist. Diesen Strom kann jeder der beispielsweise fünf Energiespeicher liefern.

Die - über die gesamte Lebensdauer des Patienten - für die Steuerung der Herzaktivität bzw. für die Therapie insgesamt notwendige Energie liegt weit unter dem Energieinhalt der Energiespeicher.

Ersichtlich hieraus wird, dass der installierte Ladungsinhalt des Energiespeichers für die Stromstärke notwendig ist, aber nicht für die Therapie. Ergo steht exorbitant Ladungsinhalt für andere Funktionen zur Verfügung und/oder der/die Energiespeicher müssen bei weitem nicht voll aufgeladen sein, um die Steuerung der Herzaktivität sicherzustellen.

In der zweiten direkten Implantationsvariante des Defibrillators oder der dritten Implantationsvariante als Herzkapsel könnten die elektrischen Parameter, Stoßspannung und Stromstärke, gleichermaßen wie vorstehend ausfallen. Hierfür besteht allerdings voraussichtlich keine Notwendigkeit, weil der Stromstoß aufgrund der Berührung, des geringeren Elektrodenabstandes und dem wesentlich geringeren Übergangswiderstand direkt ans Herz appliziert wird und keine Distanzen zum Herzen und unnötige elektrische Übergangswiederstände überwunden werden müssen.

Beispielsweise kann der/die Energiespeicher einen insgesamt geringeren Ladungsinhalt haben, bevorzugt zwischen 100 As und 250 As, wobei die gelieferte Spannung 4 V bis 8 V beträgt. Ein Mikrotransformator kann diese Spannung auf 40 V bis 80 V herauftransformieren (Übertragungsverhältnis 10). Der Stromstoß kann 0,1 A aufweisen, woraus sich ein Strom in der primärseitigen Spule des Transformators von 1 A ergibt. Dieser Strom ist für den/die Energiespeicher bedeutungslos. Der Stromstoß erfolgt bevorzugt mit 0,1 A über eine Dauer von 1ms bis 10ms.

Hinzukommt, dass die direkte Abgabe des Stromstoßes auch eine Variation einer Charakteristik des Stromstoßes zulässt. *Insoweit ist der Defibrillator bevorzugt aufgebaut, wobei die Schaltelektronik eingerichtet ist, eine Form und*/*oder Länge des Stromstoßes zu variieren.*

Beispielsweise wird die Form und/oder Länge bzw. Dauer des Stromstoßes in Abhängigkeit von individuellen Eigenschaften des Lebewesens oder in Abhängigkeit von der Art der festgestellten fehlerhaften Herzaktivität variiert.

Bevorzugt ist der Defibrillator, insbesondere in der dritten Implantationsvariante, eingerichtet, die Stromstärke des Stromstoßes zu variieren. Bevorzugt wird zuerst ein "sanfter" Stromstoß abgegeben, beispielsweise direkt aus dem/den Energiespeichern. Dieser sanfte Stromstoß kann beispielsweise bei den genannten 0,5V, 1V, 4 V bis 8 V, mit 1 mA für 3ms bis 4ms erfolgen.

Sollte dieser sanfte Stromstoß die Herzaktivität nicht wie gewünscht steuern, erhöht der Defibrillator die Stoßspannung um einen Faktor (bspw. 10), beispielsweise über den Transformator, was auch gleichermaßen zu einer entsprechenden Erhöhung des Stromes führt. Die Dauer des Stromstoßes kann vorerst gleichbleiben oder variiert werden.

Der sanfte Stromstoß wird insbesondere bei einem erkannten Herzflimmern abgegeben, um die Herzfunktion zu stabilisieren. Beispielsweise kann der Defibrillator auch Polaritäten des Herzflimmerns erkennen und eine Vielzahl des sanften Stromstoßes mit zu den Polaritäten des Herzflimmerns umgekehrten Polaritäten ausgeben, was zu einer Auslöschung des Herzflimmerns führt. *Insoweit ist der Defibrillator bevorzugt aufgebaut, wobei der Defibrillator mittels Gegenpolbestromung Fehlimpulse, z.B. eines flimmernden Herzens, zumindest teilweise auslöscht.*

*Bevorzugt weist die Schaltelektronik des Defibrillators weiterhin auf: mindestens einen Speicherungskondensator, der auf die Stoßspannung aufgeladen wird und der derart dimensioniert ist, dass er mindestens die Energie für einen Stromstoß speichert, wobei der mindestens eine Speicherungskondensator bei Auslösen durch die Schaltelektronik den Stromstoß über den Elektrodenabschnitt abgibt.*

Sofern die erläuterte Funktion der Umpolung vorgesehen ist, besitzt der Defibrillator mindestens zwei Speicherungskondensatoren, die jeweils für einen der Stromstöße mit umgekehrten Polaritäten zuständig sind.

Der erfindungsgemäße Defibrillator kann insbesondere in der zweiten direkten Implantationsvariante oder der dritten kardialen Implantationsvariante als Herzkapsel auch die Funktionen eines Herzschrittmachers übernehmen und umgekehrt.

Bevorzugt ist der erfindungsgemäße Defibrillator zur Ausführung der Funktion des Herzschrittmachers eingerichtet, die Stoßspannung durch ein Potentiometer oder durch Umkehrung einer Betriebsweise des Transformators einzustellen, insbesondere zu vermindern.

Die verminderte Stoßspannung dient insbesondere als normale Herzschrittmacherstimulation oder als sanfte Defibrillation. In den weiter unten beschriebenen Ausführungsformen wird der der verminderten Stoßspannung entsprechende Stromstoß als "sanfter Stromstoß" bezeichnet. Der sanfte Stromstoß wird bei 0,5V, 1V, 4 V oder 8V und einem sich aus dem Übergangswiederstand ergebenden Strom (bspw. 1 mA mit einer Pulslänge von 1ms bis 4ms, bevorzugt von 3ms bis 4ms), erzeugt.

In der Variante mit Potentiometer ist/sind der/die Energiespeicher über das Potentiometer mit dem Elektrodenabschnitt verbunden, beispielsweise unter Umgehung des Transformators.

In der Variante der umgekehrten Betriebsweise des Transformators kann der Transformator bidirektional betrieben werden. Die Einstellung des "sanften Stromstoßes" erfolgt, indem die von dem/den Energiespeichern gelieferte Spannung nach Wechselrichtung durch den Transformator heruntertransformiert wird. In diesem Fall übernimmt die Spule des Energieempfangsabschnitts nicht nur die erwähnte Doppelfunktion (Empfangsspule und Primärspule), sondern eine Dreifachfunktion in Form der Empfangsspule, Primärspule und nach Umkehrung der Betriebsweise des Transformators, in Form der Sekundärspule bei Reduzierung der gelieferten Spannung.

Führt der "sanfte Stromstoß" zu keiner normalen Herzfunktion, wird die Betriebsrichtung des Transformators umgekehrt und der abgegebene Stromstoß mit den im Vorhergehenden erläuterten höhere elektrischen Parametern erzeugt. In anderen Worten werden zur Erzeugung der Stromstöße die Funktionen der Primär- und Sekundärspulen des Transformators umgekehrt.

Der Defibrillator besitzt neben der bevorzugten Schaltelektronik eine Steuer-, Verarbeitungs- und Speicherelektronik, die den gesamten Defibrillator steuert und die entsprechenden zur Steuerung der Herzfunktion notwendigen Funktionalitäten realisiert. Hierzu zählen bevorzugt die im Vorhergehenden erläuterten Steuerungen der Schaltelektronik, des Transformators, der Gleichrichter, und/oder der Wechselrichter - soweit vorhanden. Darüber hinaus besitzt die Steuer-, Verarbeitungs- und Speicherelektronik bevorzugt Funktionalitäten zur Überwachung der Herzfunktion, beispielsweise Pulsmessfunktionen, Herzrhythmus-Detektionsfunktionen, Bio-Signalmessfunktionen und/oder EKG-Funktionen.

Die Steuer-, Verarbeitungs- und Speicherelektronik realisiert bevorzugt auch die Funktionen des Herzschrittmachers.

Der Energiespeicher oder die Vielzahl an Energiespeichern dienen zur Langzeitversorgung des Defibrillators, indem sie alle Komponenten, wie die Steuer-, Verarbeitungs- und Speicherelektronik mit notwendiger elektrischer Energie versorgen. Der Energieinhalt des/der Energiespeicher(s) beläuft sich auf eine solche Energiemenge, dass der Energiespeicher/die Energiespeicher den Defibrillator, bevorzugt inklusive der Herzschrittmacherfunktion, über einen Zeitraum von ein, eineinhalb oder zwei Jahren mit elektrischer Energie versorgen können, wobei eine Aufladung bevorzugt nach der jeweiligen hälftigen Zeit (0,5, 0,75 oder 1 Jahr) stattfindet.

Bei Verwendung des Defibrillators zur Entkrampfung (Herzflimmern) oder Wiederbelebung des Herzens bzw. des Herzmuskels ist es insbesondere wünschenswert, dass eine Strombahn des abgegebenen Stromstoßes möglichst weitgehend durch das Herzgewebe bzw. den Herzmuskel verläuft. Um dies zu erreichen, liefert die Erfindung *ebenfalls ein Defibrillatorsystem, das vorzugsweise mindestens zwei der im Vorhergehenden erläuterten Defibrillatoren beinhaltet, die miteinander interagieren.*

*Bevorzugt interagieren beide das Defibrillatorsystem aufbauenden Defibrillatoren, indem sie eingerichtet sind, miteinander zu kommunizieren, um beispielsweise die Abgabe der jeweiligen Stromstöße zu koordinieren. Insbesondere ist das Defibrillatorsystem eingerichtet, dass die mindestens zwei Defibrillatoren ihre Stromstöße gleichzeitig oder zu unterschiedlichen Zeitpunkten abgeben.*

Alternativ oder zusätzlich interagieren die das Defibrillatorsystem aufbauenden Defibrillatoren, indem die mindestens zwei Defibrillatoren bestimmungsgemäß so implantiert werden, dass sie zusammen ein Stromsystem bilden, bei dem zumindest Teilströme zwischen entgegengesetzten Elektroden der mindestens zwei Defibrillatoren fließen.

Hierdurch wird erreicht, dass die Strombahn zumindest eines Teilstromes möglichst weitgehend über das Herzgewebe bzw. den Herzmuskel fließt. Dieser Teilstrom ergibt sich dadurch, dass der von einem der Defibrillatoren abgegebene Stromstoß nicht nur zu seiner Gegenelektrode fließt, sondern auch zu einer Gegenelektrode des anderen Defibrillators und damit zwangsläufig über das Herzgewebe bzw. den Herzmuskel.

Das Stromsystem lässt sich bevorzugt dann realisieren, wenn die Defibrillatoren in unterschiedlichen Herzkammern sitzen und die Defibrillatoren bevorzugt räumlich möglichst so angeordnet sind, dass die entsprechenden Elektroden und Gegenelektroden linear hintereinander angeordnet sind.

Folglich ist das *Defibrillatorsystem bevorzugt eingerichtet, wobei einer der mindestens zwei Defibrillatoren bestimmungsgemäß in einer Vorkammer des Herzens implantiert wird und ein anderer der mindestens zwei Defibrillatoren in einer Hauptkammer des Herzens implantiert wird.* Die mindestens zwei das Defibrillatorsystem bildenden Defibrillatoren bilden bevorzugt gleichzeitig ein Herzschrittmachernetzwerk, das die Funktion eines Mehrkammerherzschrittmachers übernimmt. Besonders bevorzugt beinhaltet das Herzschrittmachernetzwerk die mindestens zwei das Defibrillatorsystem bildenden Defibrillatoren und eine dritte Einheit, die in eine weitere Kammer des Herzens implantiert wird. Die dritte Einheit kann ebenfalls ein Defibrillator mit den erläuterten Merkmalen sein und Teil des Defibrillatorsystems sein.

Der Aufbau des Defibrillatorsystems mit in den Herzkammern sitzenden Defibrillatoren ist (nicht zuletzt wegen den fehlenden Speicherkondensatoren) nur mit akkubetriebenen Defibrillatoren möglich. Der Aufbau mit zwei oder drei Defibrillatoren ist physikalisch der effektivste, weil er alle Möglichkeiten abdeckt (Defibrillator incl. Herzschrittmacher in beiden Kammern) und die Elektroden den empfindlichsten und wirkungsvollsten Bereich des Herzens bzw. Herzmuskels erfassen. Diese Lösung belastet das Herz am wenigsten, weil unter anderem die Stromdichte an den Polen gegenüber den konventionellen Lösungen abnimmt.

Eine weitere Lösung besteht darin nur einen Defibrillator, incl. Herzschrittmacher, an bevorzugter Stelle im Herzen, z.B. Sinusknoten, anzubringen, wobei sich die Gegenelektrode entweder in der betreffenden Herzkammer oder außerhalb des Herzens befindet.

Im Folgenden werden bevorzugte Ausführungsformen unter Bezug auf die beigefügten Figuren erläutert. Die vorstehenden allgemeinen Ausführungen in Bezug auf den erfindungsgemäßen Defibrillator gelten für die Ausführungen zu den Figuren gleichermaßen und vice versa.
**Figuren 1** zeigt einen perspektivischen Aufriss eines erfindungsgemäßen Defibrillators zur Implantation in einen Körper, wobei der Defibrillator gemäß der ersten berührungslosen Implantationsvariante oder der ersten kardialen Implantationsvariante realisiert ist.
**Figur 2** zeigt einen Längsschnitt eines erfindungsgemäßen Defibrillators, wobei der Defibrillator gemäß der dritten kardialen Implantationsvariante als Herzkapsel realisiert ist.
**Figuren 3A****,** **3B** **und** **3C** zeigen Schaltbilder des Defibrillators aus Figur 1 und Figur 2; wobei die entsprechenden Schaltbilder in der ersten indirekten Implantationsvariante, der zweiten direkten Implantationsvariante oder der dritten kardialen Implantationsvariante realisierbar sind.
**Figur 4** zeigt ein Schaltbild des Defibrillators aus Figur 1 oder Figur 2; wobei sich das Schaltbild bevorzugt für die erste indirekte oder zweite direkte Implantationsvariante des Defibrillators eignet.
**Figuren 5A bis 5C** zeigen jeweils ein Schaltbild des Defibrillators aus Figur 1; wobei das Schaltbild in der ersten indirekten Implantationsvariante oder der zweiten direkten Implantationsvariante des Defibrillators realisierbar ist.

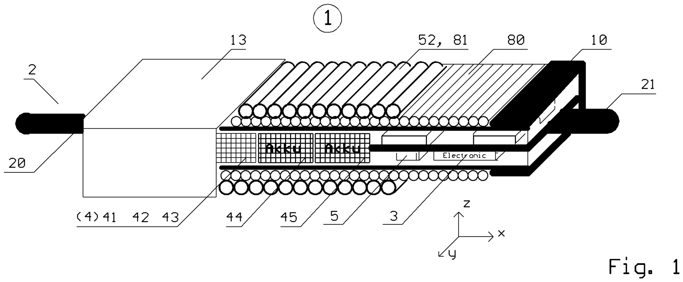

Figur 1 zeigt einen Aufbau eines erfindungsgemäßen Defibrillators 1 in einer perspektivischen Ansicht bzw. einem perspektivischen Aufriss, wobei ein Defibrillatorgehäuse 13, das die gezeigten Komponenten aufnimmt, gezeigt ist.

Der Defibrillator 1 wird bevorzugt vollständig in einen Körper eines Lebewesens, insbesondere Menschen, implantiert, wobei der Defibrillator 1 bevorzugt der ersten indirekten Implantationsvariante oder der zweiten direkten Implantationsvariante entspricht.

In dem Gehäuse 13 des Defibrillators 1 aufgenommen sind bevorzugt alle Komponenten des Defibrillators 1 bis auf einen Elektrodenabschnitt 2.

Der Elektrodenabschnitt 2 beinhaltet einen außerhalb des Gehäuses 13 zugänglichen Anschluss 20 für eine (nicht gezeigte) Kabelsonde. Zusätzlich besitzt der Elektrodenabschnitt 2 eine weitere Elektrode 21, die außen von dem Defibrillatorgehäuse 13 vorsteht und exponiert ist. Auch ist es möglich, dass eine weitere Kabelsonde bestimmungsgemäß an die Elektrode 21 anschlossen wird.

Je nachdem, ob der Defibrillator 1 gemäß der ersten indirekten oder zweiten indirekten Implantationsvariante aufgebaut ist, wird mindestens eine Kabelsonde in die Nähe des Herzens oder durch ein Gefäß hindurch ins Herz geführt.

Das Defibrillatorgehäuse 13 besitzt ein Volumen zwischen 4 cm³ und 7cm³, bevorzugt von 5 cm³ bis 6 cm³.

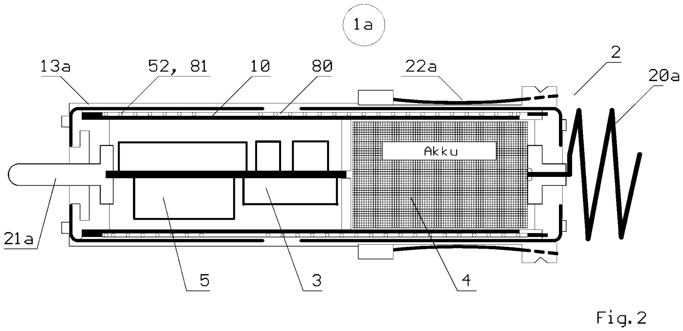

Figur 2 zeigt einen Längsschnitt eines Defibrillators 1a gemäß der der dritten Implantationsvariante.

Der Defibrillator 1a ist insbesondere eine Herzkapsel, die bestimmungsgemäß an einer Außenwand des Herzens oder innerhalb einer Herzkammer verankert wird.

Gleichermaßen wie in Figur 1 sind alle Komponenten in einem Defibrillatorgehäuse 13a aufgenommen bis auf den Elektrodenabschnitt 2.

Der Elektrodenabschnitt 2 beinhaltet eine Elektrode 20a, die als Verankerungsspirale ausgebildet ist und zur Verankerung des Defibrillators 1a in das Herzgewebe eingedreht wird. Zusätzlich beinhaltet der Elektrodenabschnitt 2 eine weitere Elektrode 21a, die außen von dem Gehäuse 13a vorsteht und exponiert ist.

Insbesondere kann diese Elektrode als elektrisch leitender, vorgespannter Draht realisiert sein, der in der Normalform in der Form eines Spazierstockes gebildet ist. Dieser Verlauf des Drahtes dient zur Verringerung des Wiederstandes zwischen den beiden Elektroden (Draht und bspw. Spirale 20a). Hierrunter ist zu verstehen, dass die weitere Elektrode 20a bzw. der Draht in Figur 2 von der dort gezeigten linken Seite aus bogenförmig in Richtung der rechten Seite des Gehäuses 13a verläuft, wobei sich ein Abstand zu dem Gehäuse 13 mit zunehmender Annäherung an die Spirale 20a leicht vergrößert. Ein der Spirale 20a zugewandtes Ende des Drahtes kann nach Verankerung des Defibrillators 1a das Herzwandgewebe berühren. Auch kann die weitere Elektrode 21a aus einer Vielzahl solcher Drähte, beispielsweise drei oder vier, gebildet sein, die bevorzugt gleichmäßig um das Gehäuse 1a herum platziert sind.

Der Defibrillator 1a gemäß Figur 2 ist wesentlich kleiner als der aus Figur 1. Das Defibrillatorgehäuse 1a besitzt ein Volumen von 0,5 cm³ bis 1,5 cm³, besonders bevorzugt von 0,6 cm³ bis 0,8 cm³.

Neben der als Elektrode 20a fungierende Verankerungsspirale kann der Defibrillator 1a noch eine weitere, zusätzliche Verankerung 22a aufweisen, die linear versetzbare Stifte beinhaltet. Bei linearer Versetzung der zusätzlichen Verankerung 22a werden die Stifte in das Herzgewebe getrieben und verlaufen bevorzugt bogenförmig. Die Stifte bzw. die zusätzliche Verankerung 22a können die Funktion der weiteren Elektrode 21a übernehmen.

Hervorzuheben ist, dass der Defibrillator 1a aus Figur 2 vollständig ohne Kabelelektroden auskommt (leadless), denen bei ungünstiger Verlegung negative Wirkungen auf das Gewebe zugeschrieben werden.

Der Defibrillator 1 aus Figur 1 beinhaltet eine Vielzahl von elektrischen Energiespeichern 41 bis 45. Die Erfindung ist hierauf nicht beschränkt. Der Defibrillator 1 kann auch nur einen einzigen elektrischen Energiespeicher 4 aufweisen, insbesondere in dem Fall, dass der Defibrillator gemäß der zweiten direkten Implantationsvariante oder der dritten kardialen Implantationsvariante realisiert ist. Figur 2 zeigt die Ausbildung des Defibrillators 1a in der dritten kardialen Implantationsvariante mit nur einem einzigen Energiespeicher 4. Prinzipiell kann auch der Defibrillator 1 in der dritten kardialen Implantationsvariante eine Vielzahl an Energiespeichern aufweisen.

Die Vielzahl der Energiespeicher ist nicht auf fünf beschränkt.

Im Folgenden werden eine Vielzahl von Schaltbildern des Defibrillators erläutert.

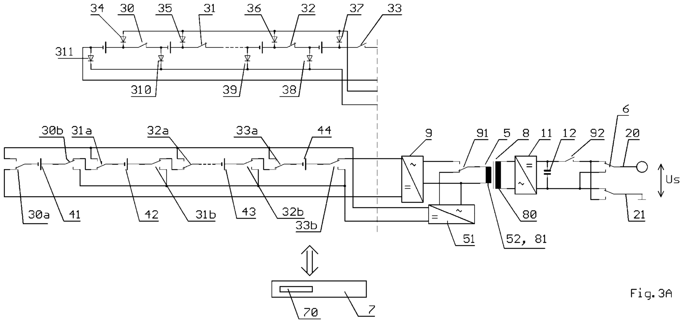

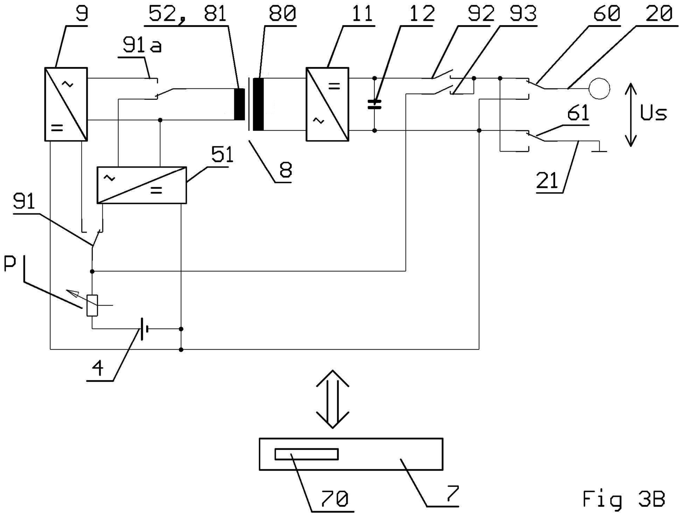

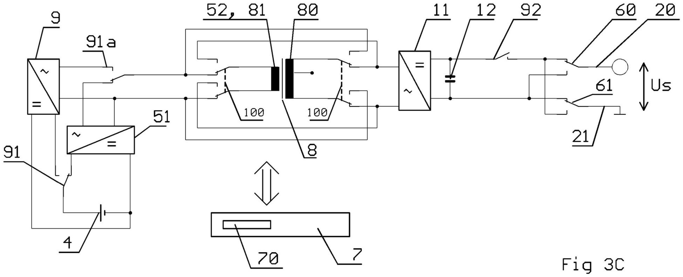

Figuren 3A, 3B und 3C zeigen Schaltbilder von möglichen Schaltungsaufbauten, gemäß denen der Defibrillator 1 in der ersten indirekten, der zweiten direkten Implantationsvariante und der dritten kardialen Implantationsvariante realisiert sein kann.

Figur 3A zeigt einen Schaltungsaufbau des Defibrillators 1, 1a mit einer Schaltelektronik 3 und einer Steuer-, Verarbeitungs- und Speicherelektronik 7.

Die Steuer-, Verarbeitungs- und Speicherelektronik 7, im Folgenden SVS-Elektronik 7 genannt, steuert die Schaltelektronik 3, was durch den Doppelpfeil angedeutet ist, und beinhaltet eine Kommunikationseinheit 70 zur Kommunikation mit beispielsweise einem Ladegerät oder einer Konfigurationseinrichtung.

Der Defibrillator 1, 1a besitzt bevorzugt die Vielzahl von Energiespeichern 41 bis 45, wobei in Figur 3A lediglich vier - aus Platzgründen - gezeigt sind. Die Energiespeicher 41 bis 45 sind bevorzugt elektrische Akkumulatoren.

Die Schaltelektronik 3 ist eingerichtet, aus der von den Energiespeichern 41 bis 45 gelieferten Spannung eine Stoßspannung Us, mit der der Stromstoß zur Steuerung der Herzaktivität abgegeben wird, zu erzeugen.

Die Schaltelektronik 3 beinhaltet eine Vielzahl von Schaltern 30a, 30b, 31a, 31b, 32a, 32b, 33a, 33b über die die SVS-Elektronik 7 die Energiespeicher 41 bis 45 parallel oder in Reihe schalten kann.

Des Weiteren beinhaltet die Schaltelektronik 3 einen Wechselrichter 9, einen Gleichrichter 51, einen Transformator 8, einen weiteren Gleichrichter 11 und eine Umpoleinrichtung 6.

Der Defibrillator 1, 1a beinhaltet darüber hinaus einen Energieempfangsabschnitt 5, der aus einer (Empfangs)Spule 52 und dem Gleichrichter 51 gebildet ist.

Bevorzugt bildet die Empfangsspule 52 gleichzeitig die primärseitigen Spule 81 des Transformators 8.

Aus Figur 1 ist ersichtlich, dass die primärseitige Spule 52, 81 bevorzugt um ein in dem Defibrillatorgehäuse 13 vorhandenes Innengehäuse 10, das bevorzugt aus einem magnetisch leitenden Material, bevorzugt ein Ferrit, gebildet ist, gewickelt ist. Das Innengehäuse 10 bildet damit einen Kern für die Spule 52, 81, der das von einem Ladegerät erzeugte magnetische Wechselfeld sammelt. Bevorzugt bildet das Innengehäuse 10 einen Kern des Transformators 8.

Eine sekundärseitige Spule 80 des Transformators 8 ist bevorzugt auch um das Innengehäuse 10 gewickelt und befindet sich bevorzugt zwischen der primärseitigen Spule 52 und dem Innengehäuse 10. Insoweit trägt die sekundärseitige Spule 80 die primärseitige Spule 81.

In Figur 2 bzw. dem Defibrillator 1a gemäß der dritten kardialen Implantationsvariante ist die Empfangsspule/primärseitige Spule 52, 81, beispielsweise neben der sekundärseitigen Spule 80 um das bevorzugt auch den Kern des Transformators 8 bildende Innengehäuse 10 gewickelt.

In beiden Figuren 1 und 2 übernehmen die Empfangsspule 52, 81 und das Innengehäuse 10 jeweils Doppelfunktionen und tragen damit erheblich zur Verringerung des Volumens des Defibrillators 1, 1a bei.

Wenn der Defibrillator 1, 1a aufgeladen wird, schaltet die SVS-Elektronik 7 die Energiespeicher 41 bis 45 parallel zueinander, indem sie die Schalter 30a, 30b, 31a, 31b, 32a, 32b, 33a, 33b entsprechend ansteuert und aus der in Figur 3A gezeigten Stellung in die jeweilige andere Stellung umschaltet.

Das von dem Ladegerät erzeugte magnetische Wechselfeld induziert eine Spannung in der Empfangsspule 52, die einen Ladestrom erzeugt. Der Gleichrichter 51 gleichrichtet den Ladestrom und führt ihn den parallelgeschalteten Energiespeichern 41 bis 45 zur Wiederaufladung zu.

Nach ausreichender Aufladung, beispielsweise vollständiger Aufladung, schaltet die SVS-Elektronik 7 in einen Betriebsmodus und schaltet hierbei die Energiespeicher 41 bis 45 in Reihe, indem sie die Schalter 30a, 30b, 31a, 31b, 32a, 32b, 33a, 33b wiederum in die in Figur 3A gezeigte Stellung zurückschaltet, und verbindet den Wechselrichter 9 durch Umschalten des Schalters 91 mit der primärseitigen Spule 81 des Transformators 8. Das Schalten der Energiespeicher 41 bis 45 in Reihe führt zu einer Vervielfachung einer von den Energiespeichern 41 bis 45 gelieferten Spannung.

In dem Betriebsmodus überwacht die SVS-Elektronik das Herz des Lebewesens, insbesondere des Menschen, und entscheidet, einen Stromstoß ST über den Elektrodenabschnitt 2 an den Körper abzugeben, wenn sie eine fehlerhafte Herzaktivität festgestellt.

Zur Erzeugung und Abgabe des Stromstoßes ST steuert die SVS-Elektronik 7 den Wechselrichter 9 an, um eine der primärseitigen Spule 81 zugeführte Wechselspannung zu erzeugen.

Der Transformator 8 transformiert diese Spannung hoch in eine an der sekundärseitigen Spule 80 ausgegebene Wechselspannung, die der weitere Gleichrichter 11, angesteuert durch die SVS-Elektronik 7, in eine Stoßspannung (Gleichspannung) gleichrichtet. Die Stoßspannung und der Stromstoß laufen bevorzugt über einen Glättungskondensator 12, sodass sie bevorzugt noch geglättet werden.

Die gleichgerichtete Stoßspannung ist gegenüber der von den Energiespeichern 41 bis 45 gelieferten Spannung vervielfacht, wobei das in Reiheschalten der Energiespeicher 41 bis 45 eine teilweise Vervielfachung darstellt und die Transformation durch den Transformator 8 eine weitere Vervielfachung.

In der bevorzugten ersten indirekten Implantationsvariante kann der Stromstoß folgende elektrischen Parameter aufweisen.

Die Stoßspannung beträgt bspw. +/- 400 V und treibt den über den Elektrodenabschnitt abgegebenen Stromstoß mit einer Stärke von 1A. Diese Ströme können die elektrischen Energiespeicher 41 bis 45 liefern. Beispielsweise besitzt jeder einen Ladungsinhalt von 500As und eine Entnahmerate von 40 - 50 mA/As. Folglich ist jeder Energiespeicher 41 bis 45 in der Lage einen Strom zwischen 20 - 25 A zu liefern.

Jeder Energiespeicher 41 bis 45 liefert außerdem eine Spannung von bspw. 4V, weshalb sich durch die Reihenschaltung an der primärseitigen Spule 52, 81 eine Spannung von 20 V ergibt.

Bei einem beispielhaften Übertragungsverhältnis des Transformators von 20 und einer sich damit ergebenden sekundärseitigen Spannung von 400 V, beträgt der erforderliche primärseitige Strom 20 A.

Ein in Figur 3A gezeigter optionaler Schalter 92 ist bei Abgabe des Stromstoßes geschlossen.

Hingegen können in der ersten direkten Implantationsvariante oder der dritten kardialen Implantationsvariante aus Figur 2 die elektrischen Parameter, Gleichspannung Us und Gleichstromstärke des Stromstoßes ST, wesentlich geringer ausfallen, da der abgegebene Stromstoß unmittelbar und direkt auf das Herz wirkt und der dort vorliegende Übergangswiderstand geringer ist, verglichen mit einem Übergangswiderstand, den der Defibrillator 1 in der ersten berührungslosen Implantationsvariante durch die obigen elektrischen Parametern überwinden muss. Auch wird davon ausgegangen, dass der Energieinhalt des Stromstoßes ST geringer ausfallen kann.

Beispielsweise liegt die Stoßspannung Us (Gleichspannung) zwischen 50 V und 150 V, insbesondere zwischen 50 V und 80 V, und die Stromstärke des Stromstoßes bei beispielsweise 0,1A bis 1A. Den Ladungsinhalt des Stromstoßes ST steuerte SVS-Elektronik 7 durch Steuerung der Pulslänge PL (bspw. 1ms bis 10ms).

Vor diesem Hintergrund kann notwendige Ladungsinhalt der Energiespeicher 41 bis 45 potenziell verringert werden, wenn der Schaltungsaufbau für die erste kardiale Implantationsvariante oder die weitere Implantationsvariante eingesetzt wird. Beispielsweise kann der Ladungsinhalt der Energiespeicher auf 100 As bis 250 As verringert werden. Auch ist es möglich, die Anzahl der Energiespeicher 41 bis 45 zu verringern, beispielsweise nur einen einzigen Energiespeicher vorzusehen, wie es in Figur 2 gezeigt ist. Die Schalter 30a, 30b, 31a, 31b, 32a, 32b, 33a, 33b verringern sich dementsprechend.

Figur 3A zeigt im oberen Teil eine Modifikation der Schaltelektronik 3, die darin besteht, dass für die Vervielfachung der Spannung der Energiespeicher 41 - 45 Dioden 34, 35, 36, 37, 38, 39, 310, 311, die beispielsweise einfache PN-Dioden sein können, zum Einsatz kommen. Hierdurch lässt sich die Anzahl der Schalter 30a, 30b, 31a, 31b, 32a, 32b, 33a, 33b um 50% halbieren, sodass lediglich die gezeigten Schalter 30, 31, 32, 33 verbleiben. Der verbleibende Teil des Schaltungsaufbaus (rechts der gestrichelten Linie) ist identisch mit dem bereits erläuterten Schaltungsaufbau, weshalb er in Kontext mit der Modifikation nicht gezeigt ist.

Die erläuterte Abgabe des Stromstoßes erfolgt, wie aus den obigen Erläuterungen verständlich ist, gemäß Figur 3A direkt aus den Energiespeichern 41 bis 45, lediglich unter bevorzugter Transformation und Glättung, ohne dass ein Speicherungskondensator mit der für den Stromstoß notwendigen Energie vor Abgabe aufgeladen werden muss.

Das hat erhebliche Vorteile, die beispielsweise darin bestehen, dass das Volumen des Defibrillators 1, 1a ohne Speicherungskondensatoren erheblich geringer ausfallen kann und mehrere Stromstöße ohne - durch für die Zwischenspeicherung notwendige Zeit - beschränkt zu sein, abgegeben werden können.

Außerdem lässt sich die Ladungsmenge des Stromstoßes ST durch Variation der Pulslänge PL ändern.

Die SVS-Elektronik 7 ist bevorzugt eingerichtet, die Form und/oder insbesondere die Dauer bzw. Pulslänge PL des Stromstoßes zeitlich zu variieren, beispielsweise in Abhängigkeit von individuellen Eigenschaften des Lebewesens oder in Abhängigkeit von der Art der festgestellten fehlerhaften Herzaktivität.

Die Umpolungseinrichtung 6 ermöglicht eine Umpolung aufeinanderfolgender Stromstöße. Das erfolgt beispielsweise dadurch, dass die SVS-Elektronik 7 zwei Schalter 60, 61 der Umpoleinrichtung entsprechend ansteuert. Der Umpolung der Stromstöße werden aktivierende Eigenschaften zugesprochen.

Figur 3B zeigt ein weiteres Schaltbild eines Schaltungsaufbaus, gemäß dem der Defibrillator 1, 1a aufgebaut sein kann.

Der gezeigte Schaltungsaufbau eignet sich für alle Implantationsvarianten, insbesondere allerdings für die zweite direkte Implantationsvariante aus Figur 1 und die dritte kardiale Implantationsvariante gemäß Figur 2.

Der in Figur 2 gezeigte Schaltungsaufbau beinhaltet nur einen einzigen Energiespeicher 4, der beispielsweise einen Ladungsinhalt von 250 As aufweist.

Alternativ kann der Defibrillator 1 wie in Figur 1 gezeigt die Vielzahl an Energiespeichern 41 bis 45 beinhalten. Sofern der Defibrillator 1 die Vielzahl an Energiespeichern 41 bis 45 besitzt, können diese wiederum für die Aufladung parallel und für den Betrieb des Defibrillators 1, 1a in Reihe geschaltet werden.

Die folgende Beschreibung geht zur Vereinfachung von einem einzigen Energiespeicher 4 aus.

Wenn der Defibrillator 1, 1a mit dem Schaltungsaufbau gemäß Figur 3B aufgeladen wird, schaltet die SVS-Elektronik 7 den Schalter 91 in die in Figur 3B gezeigte Stellung.

Ein von dem Ladegerät erzeugtes magnetisches Wechselfeld induziert eine Spannung in der Empfangsspule 52, die daraufhin einen Ladestrom erzeugt und abgibt.

Der Gleichrichter 51 gleichrichtet den Ladestrom und führt ihn dem Energiespeicher 4 zur Wiederaufladung zu.

Nach ausreichender Aufladung, beispielsweise vollständiger Aufladung, schaltet die SVS-Elektronik 7 in den Betriebsmodus, indem sie die Schalter 91, 91a umschaltet und damit den Energiespeicher 4 mit dem Wechselrichter 9 sowie den Wechselrichter 9 mit der primärseitigen Spule 81 des Transformators 8 verbindet.

In dem Betriebsmodus überwacht die SVS-Elektronik 7 das Herz des Lebewesens, insbesondere des Menschen, und entscheidet, den Stromstoß ST über den Elektrodenabschnitt 2 an den Körper abzugeben, wenn sie eine fehlerhafte Herzaktivität festgestellt.

Zur Erzeugung und Abgabe des Stromstoßes ST steuert die SVS-Elektronik 7 den Wechselrichter 9 an, um eine der primärseitigen Spule 81 zugeführte Wechselspannung zu erzeugen. Der Transformator 8 transformiert diese Spannung hoch in eine an der sekundärseitigen Spule 80 ausgegebene Wechselspannung, die der weitere Gleichrichter 11, angesteuert durch die SVS-Elektronik 7, in die hochfrequent pulsierende Gleichspannung zur Stoßspannung gleichrichtet und geglättet. Die Stoßspannung (Us) und der Stromstoß erfolgen bevorzugt über den Glättungskondensator 12. Die Frequenz liegt bevorzugt zwischen 100kHz und 200kHz.

Bei Abgabe des Stromstoßes über den Transformator 8 ist der Schalter 92 geschlossen und ein weiterer Schalter 93 geöffnet.

Der Energiespeicher 4 liefert beispielsweise 4 V oder 8V.

Beispielsweise liegt die Stoßspannung Us (Gleichspannung) zwischen 40 V und 150 V, insbesondere zwischen 40 V und 80 V, und die Stromstärke des Stromstoßes bei beispielsweise 0,1 A. Den Ladungsinhalt des Stromstoßes ST steuert die SVS-Elektronik 7 durch Steuerung der Pulslänge PL.

Der Defibrillator 1, 1a mit dem Schaltungsaufbau gemäß Figur 3B besitzt bevorzugt eine zusätzliche Funktion, dass der Stromstoß ST direkt aus dem Energiespeicher 4 an das Herz abgegeben werden kann. Dieser Stromstoß ist ein "sanfter Stromstoß", wie zum Beispiel bei Anregung als "normaler" Herzschrittmacher. Der entsprechende sanfte Stromstoß erfolgt bei niedriger Spannung, beispielsweise 0,5V, 1V, 4 V oder 8V und einem sich aus dem Übergangswiederstand ergebenden Strom (bspw. 1 mA mit einer Pulslänge von 1ms bis 4ms, bevorzugt von 3ms bis 4ms). Nachdem der Übergangswiederstand im/am Herzen gering ausfällt, stellt sich eine die Herzaktivität steuernde Stromstärke und Wirkung ein.

In dem Fall der Abgabe des sanften Stromstoßes verbleibt der Schalter 92 offen oder die SVS-Elektronik 7 öffnet den Schalter 92 vorab.

Den Strang über den Wechselrichter 9, den Transformator 8 und den Gleichrichter 11 betreibt die SVS-Elektronik 7 ergo nicht.

Hingegen steuert die SVS-Elektronik 7 für die Abgabe des sanften Stromstoßes den Schalter 93 derart an, dass der Stromstoß ST mit der gewünschten Pulslänge PL direkt aus dem Energiespeicher 4 an das Herz abgegeben wird. Über ein optionales elektronisches Potentiometer P kann die SVS-Elektronik 7 die Stoßspannung Us und bevorzugt die Herzschrittmacherspannung regelbar beeinflussen.

Die Funktion der Umpoleinrichtung 6 entspricht der aus Figur 3A, wobei auf entsprechende Ausführungen verwiesen wird.

Der Defibrillator 1, 1a mit dem Schaltungsaufbau gemäß Figur 3B ermöglicht insbesondere eine sehr schonende Steuerung der Herzaktivität.

Wenn der Defibrillator 1a bzw. die SVS-Elektronik 7 beispielsweise ein Herzflimmern erkennt, kann eine Vielzahl des sanften Stromstoßes direkt aus dem Energiespeicher 4 hintereinander abgeben werden, indem die SVS-Elektronik 7 den Schalter 93, wie erläutert, angesteuert.

Bevorzugt kann die SVS-Elektronik 7 die sanften Stromstöße durch Ansteuerung der Umpoleinrichtung 6 mit umgekehrten Polaritäten ausgeben.

Besonders bevorzugt ist die SVS-Elektronik 7 eingerichtet, Polaritäten von durch das Herzflimmern erzeugten elektrischen Bio-Signalen des Herzens zu erkennen und die sanften Stromstöße mit jeweils das Herzflimmern auslöschenden Polaritäten auszugeben.

Wenn die SVS-Elektronik 7 erkennt, dass die über den Schalter 93 ausgegebenen sanften Stromstöße nicht ausreichen, die Herzaktivität in einer gewünschten Weise zu steuern, nimmt die SVS-Elektronik 7 den Strang über den Wechselrichter 9, den Transformator 8 und den Gleichrichter 11 in Betrieb, sodass der Stromstoß oder die Stromstöße mit den oben angegebenen höheren Spannungen und Strömen abgegeben werden.

Im Falle eines durch die SVS-Elektronik 7 erkannten Herzstillstandes, nimmt die SVS-Elektronik 7 unmittelbar den Strang über den Wechselrichter 9, den Transformator 8 und den Gleichrichter 11 in Betrieb.

Wenngleich nicht auf den Schaltungsaufbau aus Figur 3B eingeschränkt, eignet sich dieser überaus dafür, dass der Defibrillator 1a auch die Funktion eines Herzschrittmachers übernimmt. Oder anders ausgedrückt lässt sich mit diesem Schaltungsaufbau ein in das Herz implantierbarer aufladbarer Herzschrittmacher mit Defibrillator realisieren.

Der sanfte Stromstoß entspricht einem durch die Herzschrittmacherfunktion erzeugten Stromstoß zur Anregung eines ausbleibenden Herzschlages. Insoweit kann die Herzschrittmacherfunktion auch für den Defibrillator genutzt werden, insbesondere bei Herzflimmern, wobei der Strang über den Wechselrichter 9, den Transformator 8 und den Gleichrichter 11 die oben beschriebene Funktion des Defibrillators 1a übernimmt.

Figur 3C zeigt eine bevorzugte Variante des Schaltungsaufbaus aus Figur 3B für den entsprechend bevorzugten Defibrillator 1, 1a.

Bis auf die zusätzliche Funktion, dass der sanfte Stromstoß abgegeben werden kann, sind die Funktionen des Schaltungsaufbaus aus Figur 3C, insbesondere die Aufladung und die Abgabe des Stromstoßes ST in dem Betriebsmodus über den Transformator 8, mit denen aus Figur 3B identisch. Insoweit wird auf die entsprechenden Ausführungen verwiesen.

Anders hingegen ist die Realisierung der Funktion der Abgabe des sanften Stromstoßes. Das im Vorhergehenden erwähnte Potentiometer P und die entsprechende Kontaktierung mit der Umpoleinrichtung 6 sind nicht notwendig.

Die Funktion des Potentiometers P zur Anpassung/Beinflussung der Stoßspannung Us und bevorzugt der Herzschrittmacherspannung übernimmt der Transformator 8. Hierfür besitzt der Transformator 8 zwei Schalteinrichtungen 100 und Bypass-Kontaktierungen, die einen bidirektionalen Betrieb des Transformators 8 ermöglichen.

Figur 3C zeigt den Schaltungsaufbau bei Aufladung des Energiespeichers 4. Die Schalter 91a, 91 sind so geschaltet, dass die von der Empfangsspule 52 empfangene Energie über den Gleichrichter 51 dem Energiespeicher 4 zugeführt wird. Auf die entsprechenden Ausführungen zur Figur 3B wird verwiesen.

Nach Aufladung schalten die Schalter 91, 91a in die jeweilige andere Schaltposition, sodass der Energiespeicher 4 über den Wechselrichter 9 mit der Primärseite bzw. der Primärspule 81 des Transformators 8 verbunden ist. In dieser Schaltstellung befindet sich der Defibrillator 1, 1a in dem Betriebsmodus, wobei der Defibrillator 1, 1a bei Bedarf, gesteuert durch die SVS-Elektronik 7, über den Strang aus Wechselrichter 9, Transformator 8, Gleichrichter 11, bevorzugten Glättungskondensator 12, und die bevorzugte Umpoleinrichtung 6 den Stromstoß ST aus dem Energiespeicher 4 an das Herz abgeben kann.

Wenn der Defibrillator 1, 1a die Funktion des sanften Stromstoßes mit den elektrischen Parametern, wie sie unter Figur 3B genannt wurden, ausführt, schaltet die SVS-Elektronik 7 die Schalteinrichtungen 100 jeweils in die gegenüber Figur 3C andere Schaltstellung, wobei Schalter 91, 91a unverändert bleiben.

Das führt dazu, dass der Transformator 8 umgekehrt betrieben wird und die Spule 80 die Primärspule und die Spule 81 die Sekundärspule bildet. Damit transformiert der Transformator 8 die von dem Wechselrichter 9 abgegebene Wechselspannung herunter, sodass der sanfte Stromstoß ST von der in diesem Fall als Sekundärspule fungierenden Spule 81 über den Gleichrichter 11, bevorzugten Glättungskondensator 12 und bevorzugten Umpoleinrichtung 6 an das Herz abgegeben wird. Insoweit übernimmt die Spule 81 gegenüber den vorstehenden Ausführungsformen nicht nur eine Doppelfunktion als Empfangsspule 52 und Primärspule 81, sondern eine Dreifachfunktion als Empfangsspule 52, Primärspule 81 und nach Umschalten der Schalteinrichtungen 100, als Sekundärspule für die Spannungsverminderung.

Dieser so erzeugte "sanfte Stromstoß" dient, wie bereits unter Figur 3B genannt, als Anregung wie bei einem "normalen" Herzschrittmacher oder als sanfte Defibrillation. Der entsprechende sanfte Stromstoß erfolgt bei niedriger Spannung, beispielsweise 0,5V, 1V, 4 V oder 8V und einem sich aus dem Übergangswiederstand ergebenden Strom (bspw. 1 mA mit einer Pulslänge von 1ms bis 4ms, bevorzugt von 3ms bis 4ms).

Führt die Abgabe des sanften Stromstoßes ST nicht zum Erfolg, schaltet die SVS-Elektronik 7 die Schalteinrichtungen 100 wieder um, sodass der Stromstoß ST mit den erläuterten höheren elektrischen Parametern abgegeben wird. Diesbezüglich wird auf die entsprechenden Ausführungen zu Figur 3B verwiesen.

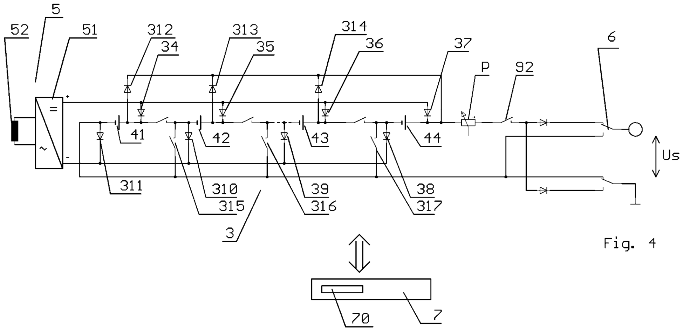

Figur 4 zeigt einen weiteren Schaltungsaufbau, gemäß dem der Defibrillator 1, 1a aufgebaut sein kann.

Der entsprechende Schaltungsaufbau eignet sich insbesondere für den Defibrillator 1, 1a gemäß der zweiten direkten Implantationsvariante oder der dritten kardialen Implantationsvariante in Form der Herzkapsel.

Der gezeigte Schaltungsaufbau kommt ohne die Wicklung 80 aus.

Die Empfangsspule 52 des Energieempfangsabschnitts 5 übernimmt keine Doppelfunktion, sondern dient ausschließlich für den Empfang der zur Aufladung der Energiespeicher 41 bis 45 notwendigen Energie, die das Ladegerät durch Erzeugung des magnetischen Wechselfeldes überträgt.

Der Gleichrichter 51 des Energieempfangsabschnitts wird durch die SVS-Elektronik 7 zur Gleichrichtung der von der Empfangsspule 52 ausgegebenen Wechselspannung betrieben.

Die Energiespeicher 41 bis 45 können durch die Schaltelektronik 3 zur Wiederaufladung parallel und für den Betrieb des Defibrillators in Reihe geschaltet werden.

Die Schaltelektronik 3 besitzt hierfür den gleichen Aufbau wie die Modifikation, die im oberen Teil der Figur 3A gezeigt ist. Diesbezüglich wird auf die entsprechenden Ausführungen verwiesen.

Prinzipiell kann auch der Defibrillator 1 gemäß der ersten indirekten Implantationsvariante mit dem gezeigten Schaltungsaufbau realisiert sein, wobei in diesem Fall - auf Kosten des Volumens - die Vielzahl an Energiespeichern so viele Energiespeicher besitzt, dass sich in der Reihenschaltung eine gelieferte vervielfachte Spannung von beispielsweise 400 V ergibt.

Zusätzlich beinhaltet der Schaltungsaufbau die zusätzlichen Dioden 312, 313, 314 und die Schalter 315, 316, 317, wobei die SVS-Elektronik 7 durch diese Dioden und Schalter einzelne der Energiespeicher 41 bis 45 in Figur 4 entweder von rechts oder von links beginnend aus der Reihenschaltung ausschließen kann, um damit die Stoßspannung Us des abgegebenen Stromstoßes zu ändern. Über ein optionales elektronisches Potentiometer P kann die SVS-Elektronik 7 die Stoßspannung Us und bevorzugt die Herzschrittmacherspannung regelbar beeinflussen. Ein solches elektronisches Potentiometer P kann in allen in den Figuren 3A, 3B und 4 gezeigten Schaltungsaufbauten vorhanden sein.

Allgemein kann auch der Defibrillator 1, 1a mit einem Schaltungsaufbau aus Figuren 3A und 3B die Funktion des Herzschrittmachers übernehmen. Zur Abgabe des sanften Stromstoßes (bzw. einer Vielzahl solcher sanften Stromstöße) besitzen die entsprechenden Schaltungsaufbauten geeignete Einstellfunktionen zur Einstellung der Stoßspannung und Stromstärke des sanften Stromstoßes

Wenngleich die bislang erläuterten Schaltungsaufbauten besonders bevorzugt keine Speicherkondensatoren aufweisen und diese auch nicht erfordern, weil die aus den Energiespeichern entnehmbaren Ströme ausreichend hoch sind, ist die Erfindung hierauf nicht beschränkt.

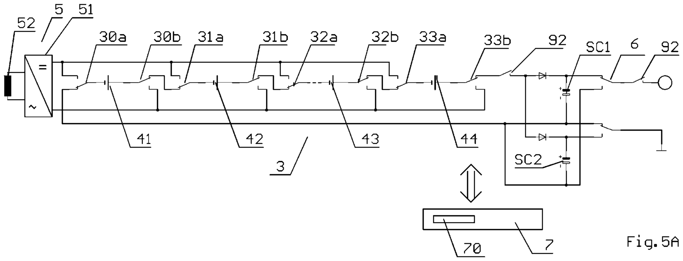

Die Figuren 5A bis 5C zeigen Schaltungsaufbauten mit Speicherkondensatoren. Insbesondere der Defibrillator 1 gemäß der ersten indirekten Implantationsvariante und der zweiten direkten Implantationsvariante kann gemäß den gezeigten Schaltungsaufbau aufgebaut sein.

Der Schaltungsaufbau gemäß Figur 5A kommt ohne die Wicklung 80 aus. Insbesondere der Defibrillator 1 gemäß der zweiten direkten Implantationsvariante kann diesen Schaltungsaufbau besitzen. Sofern der Defibrillator 1 gemäß der ersten indirekten Implantationsvariante realisiert werden soll, erhöht sich die Anzahl der Energiespeicher soweit, dass sich in Reihenschaltung eine vervielfachte Spannung von beispielsweise 400 V ergibt.

Der Energieempfangsabschnitt 5 hat denselben Aufbau wie in Figur 4, auf die entsprechenden Ausführungen wird verwiesen.

Die Schaltelektronik 3 hat hingegen denselben Aufbau wie aus Figuren 3A (unterer Teil), insoweit wird auf die dortigen Ausführungen verwiesen.

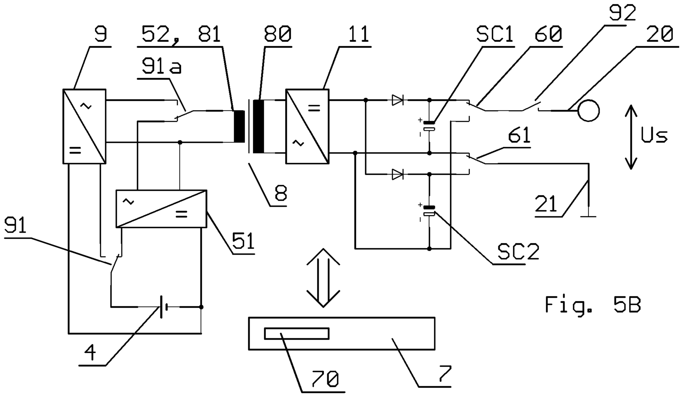

Figur 5B zeigt einen Schaltungsaufbau, gemäß dem der Defibrillator gemäß der ersten indirekten oder zweiten direkten Implantationsvariante aufgebaut sein kann. Die Funktionsweise ist mit der aus Figur 3B identisch, mit dem Unterschied, dass zum einen die zusätzliche Funktion der direkten Abgabe des Stromstoßes nicht vorgesehen ist und zum anderen die Speicherkondensatoren vorgesehen sind.

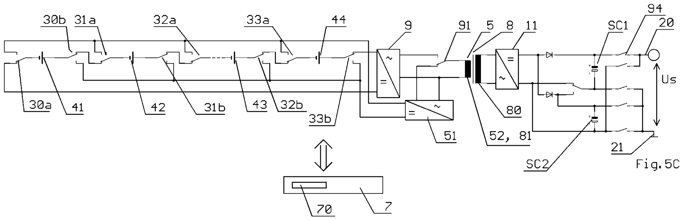

Der Schaltungsaufbau gemäß Figur 5C ist identisch mit dem aus Figur 3A (unterer Teil). Insoweit wird auf die entsprechenden Ausführungen verwiesen. Unterschiedlich ist lediglich, dass der ohnehin optionale Schalter 92 nicht vorhanden ist und die Speicherkondensatoren SC1/SC2 vorgesehen sind.

Die Schaltungsaufbauten aus Figuren 5A bis 5C besitzen jeweils insgesamt zwei Speicherungskondensatoren SC1 und SC2, wobei jeder die gesamte Energie für einen Stromstoß speichert. Die von den Speicherungskondensatoren abgegebenen Stromstöße haben zueinander umgekehrte Polaritäten, wobei die Umpoleinrichtung 6 zwischen den Speicherungskondensatoren umschalten kann. Die Abgabe des/der Stromstöße steuert die SVS-Elektronik 7 durch den/die gezeigten Schalterkombination 94. Der Schaltungsaufbau aus Figur 5C kann darüber hinaus die doppelte Stoßspannung erzeugen, jedoch nur einpolig.

## Patentansprüche

1. Defibrillator (1) zur Implantation in einen Körper eines Lebewesens und zur Steuerung einer Herzaktivität des Herzens des Lebewesens, aufweisend:
einen Elektrodenabschnitt (2), der bestimmungsgemäß bei Implantation des Defibrillators derart angeordnet wird, dass ein über den Elektrodenabschnitt (2) abgegebener Stromstoß (ST) die Herzaktivität steuert
eine mit dem Elektrodenabschnitt (2) verbundene Schaltelektronik (3), die eingerichtet ist, den Defibrillator zu steuern und die Abgabe des Stromstoßes (ST) auszulösen;
mindestens einen elektrischen Energiespeicher (4), der eine Spannung zur Langzeitversorgung liefert, wobei
die Schaltelektronik (3) eingerichtet ist, aus der gelieferten Spannung eine Stoßspannung, mit der der Stromstoß (ST) zur Steuerung der Herzaktivität abgegeben wird, zu erzeugen, bevorzugt die gelieferte Spannung zur Erzeugung der Stoßspannung zu vervielfältigen und/oder zu reduzieren; und
einen mit der Schaltelektronik (3) verbundenen Energieempfangsabschnitt (5), der derart eingerichtet ist, dass er Energie kontaktlos empfangen und an den Energiespeicher (4) zum Wiederaufladen des Energiespeichers (4) abgeben kann, wobei der Energieempfangsabschnitt (4) zumindest eine Spule (52) aufweist, die eingerichtet ist, zumindest die Energie zu empfangen und über einen Gleichrichter (51) an den Energiespeicher (4) abzugeben, wenn sie von einem magnetischen Wechselfeld, das ein externes Ladegerät erzeugt, durchsetzt wird.

2. Defibrillator nach Anspruch 1, wobei die Schaltelektronik (3) eingerichtet ist, die Stoßspannung bei Abgabe des Stromstoßes (ST) oder die Stoßspannung aufeinanderfolgender Stromstöße vorzugsweise umzupolen, um so eine Stromrichtung des Stromstoßes (ST) zu ändern.

3. Defibrillator nach Anspruch 1 oder 2, wobei eine Steuer-, Verarbeitungs- und Speicherelektronik (7) eine Kommunikationseinheit (70) aufweist, die eingerichtet ist, zumindest mit dem externen Ladegerät zur Ausrichtung und/oder Veränderung des magnetischen Wechselfeldes zu kommunizieren, um eine für das Wiederaufladen erforderliche Zeit zu vermindern.

4. Defibrillator nach einem der Ansprüche 1 bis 3, aufweisend:
eine Vielzahl von elektrischen Energiespeichern (41 - 45), die die Spannung zur Langzeitversorgung liefern, wobei
die Schaltelektronik (3) eingerichtet ist, die Energiespeicher (41 - 45) für das Wiederaufladen parallel zu schalten und, um die Vervielfachung der Spannung zumindest teilweise durchzuführen, in Reihe zu schalten.

5. Defibrillator nach einem der Ansprüche 1 bis 4, wobei
die Schaltelektronik einen Transformator (8) aufweist, der die zur Langzeitversorgung gelieferte oder teilweise vervielfachte Spannung zur Erzeugung der Stoßspannung vervielfacht und/oder reduziert.

6. Defibrillator nach Anspruch 5, wobei
die zur Langzeitversorgung gelieferte oder teilweise vervielfachte Spannung über einen Wechselrichter (9) der Schaltelektronik (3) an einer primärseitigen Spule (52, 81) des Transformators (8) anliegt, und nach Gleichrichtung
die Stoßspannung an einer sekundärseitigen Spule (80) des Transformators (8) ausgegeben wird.

7. Defibrillator nach einem der Ansprüche 5 und 6, wobei
die Spule (52, 81) des Energieempfangsabschnitts (5) eine Doppelfunktion übernimmt, indem die Spule für das Wiederaufladen des/der Energiespeicher(s) eine Empfangsspule (52) bildet und für die Vervielfachung der zur Langzeitversorgung gelieferten oder teilweise vervielfachten Spannung eine primärseitige Spule (81) des Transformators (8) bildet.

8. Defibrillator nach Anspruch 7 in Rückbezug auf Anspruch 6, wobei
der Wechselrichter (9) der Schaltelektronik (3) ein bidirektionaler Wechsel-/Gleichrichter ist, der
(i) zum einen bei dem Wiederaufladen eine von der Empfangsspule (52) ausgegebene Ladewechselspannung in eine Ladegleichspannung gleichrichtet und dem/den Energiespeicher(n) (4) zuführt, und
(ii) zum anderen bei Erzeugung der Stoßspannung die zur Langzeitversorgung gelieferte oder teilweise vervielfachte Spannung wechselrichtet und der primärseitigen Spule (81) des Transformators (8) zuführt.

9. Defibrillator nach Anspruch 7 in Rückbezug auf Anspruch 6, wobei
die Schaltelektronik (3) neben dem Wechselrichter (9), der bei Erzeugung der Stoßspannung die zur Langzeitversorgung gelieferte oder teilweise vervielfachte Spannung wechselrichtet und der primärseitigen Spule (81) des Transformators (8) zuführt, einen gesonderten Gleichrichter (51) aufweist, der bei dem Wiederaufladen eine von der Empfangsspule (52) ausgegebene Ladewechselspannung in eine Ladegleichspannung gleichrichtet und dem/den Energiespeicher(n) (4) zuführt.

10. Defibrillator nach einem der vorangehenden Ansprüchen 1 bis 9, wobei
die Spule (52, 81) des Energieempfangsabschnittes (5) einen magnetisch leitenden Kern (10), bevorzugt einen Ferrit, beinhaltet.

11. Defibrillator nach Anspruch 10, wobei
der Kern (10) einen Transformatorkern (82) des Transformators (8) umschließt.

12. Defibrillator nach Anspruch 11, wobei
der Transformator (8) ein Kerntransformator, bevorzugt ein Ringkerntransformator oder Topfkerntransformator oder Hohlzylinderkerntransformator ist.

13. Defibrillator nach einem Anspruch 10, 11 oder 12, wobei
der Kern (10) ein Gehäuse bildet, in dem die Steuer-, Verarbeitungs- und Speicherelektronik (7) und/oder Schaltelektronik, und der/die Energiespeicher aufgenommen sind und auf das die Spule (52, 81) des Energieempfangsabschnittes (5) gewickelt ist.

14. Defibrillator nach Anspruch 13, wobei
das den Kern (10) bildende Gehäuse einen Transformatorkern (82) des Transformators (8) bildet, und
die die Stoßspannung ausgebende sekundärseitige Spule (80) des Transformators (8) auf das Gehäuse (10) gewickelt ist und bevorzugt die Ladespule trägt.

15. Defibrillator gemäß einem der vorgehenden Patentansprüche 1 bis 14, wobei
bei Auslösung durch die Schaltelektronik (3)
die Stoßspannung über den Elektrodenabschnitt (2) ausgegeben wird und der Stromstoß (ST) aus dem/den Energiespeichern (4) oder von der sekundärseitigen Spule (80) des Transformators über den Elektrodenabschnitt (2) zur Steuerung der Herzaktivität direkt, lediglich unter Glättung abgegeben wird.

16. Defibrillator nach Anspruch 15, wobei
die Schaltelektronik (3) eingerichtet ist, eine Form und/oder Länge (PL) des Stromstoßes (ST) zu variieren.

17. Defibrillator nach einem der vorangehenden Ansprüche1 bis 15, wobei die Schaltelektronik (3) weiterhin aufweist:
mindestens einen Speicherungskondensator (13), der auf die Stoßspannung aufgeladen wird und der derart dimensioniert ist, dass er mindestens die Energie für einen Stromstoß (ST) speichert, wobei
der mindestens eine Speicherungskondensator (13) bei Auslösen durch die Schaltelektronik den Stromstoß (ST) über den Elektrodenabschnitt (2) abgibt.

18. Defibrillator nach einem der Ansprüche 1 bis 17, wobei der Defibrillator zusätzlich eine Funktion eines Herzschrittmachers mit übernimmt.

19. Defibrillator nach Anspruch 18, wobei der Defibrillator zur Ausführung der Funktion des Herzschrittmachers eingerichtet ist, die Stoßspannung durch ein Potentiometer oder durch Umkehrung einer Betriebsweise des Transformators 8 einzustellen.

20. Defibrillator nach einem der Ansprüche 1 bis 19, wobei der Defibrillator mittels Gegenpolbestromung Fehlimpulse, z.B. eines flimmernden Herzens, zumindest teilweise auslöscht.

21. Defibrillatorsystem umfassend mindestens zwei der Defibrillatoren gemäß einem der voranstehenden Ansprüche 1 bis 20, wobei beide Defibrillatoren eingerichtet sind, miteinander zu kommunizieren.

22. Defibrillatorsystem gemäß Patentanspruch 21, wobei einer der mindestens zwei Defibrillatoren bestimmungsgemäß in einer Vorkammer des Herzens implantiert wird und ein anderer der mindestens zwei Defibrillatoren in einer Hauptkammer des Herzens implantiert wird.

23. Defibrillatorsystem gemäß Patentanspruch 21 oder 22, wobei die mindestens zwei Defibrillatoren ihre Stromstöße (ST) gleichzeitig oder zu unterschiedlichen Zeitpunkten abgeben.

24. Defibrillatorsystem gemäß einem der Patentansprüche 21 bis 23, wobei die mindestens zwei, bevorzugt drei Defibrillatoren bestimmungsgemäß so implantiert werden, dass sie zusammen ein Stromsystem bilden, bei dem zumindest Teilströme zwischen entgegengesetzten Elektroden der mindestens zwei Defibrillatoren fließen.
